# EUROPEAN PATENT SPECIFICATION

(11) **EP 2 225 563 B1**
(45) Date of publication and mention of the grant of the patent: **21.01.2015**
(21) Application number: 08855440.7
(22) Date of filing: 20.10.2008
(51) Int. Cl.: G01N 33/574

(54) **AUTOMATED ENUMERATION AND CHARACTERIZATION OF CIRCULATING MELANOMA CELLS IN BLOOD**
AUTOMATISCHE ZÄHLUNG UND CHARAKTERISIERUNG VON IM BLUT ZIRKULIERENDEN MELANOMZELLEN
DÉNOMBREMENT ET CARACTÉRISATION AUTOMATISÉS DE CELLULES DE MÉLANOME CIRCULANTES DANS LE SANG

(30) Priority: 27.11.2007 US 4345 P
(43) Date of publication of application: 08.09.2010
(73) Proprietor: Janssen Diagnostics, LLC, Raritan, NJ 08869 (US)
(72) Inventor: CONNELLY, Mark, Carle, Doylestown, PA 18901 (US); RAO, Galla, Chandra, Princeton Junction, NJ 08550 (US); TERSTAPPEN, Leon, NL-1095 DW Amsterdam (NL)
(74) Representative: Goodfellow, Hugh Robin
(86) International application number: PCT/US2008/080436
(87) International publication number: WO 2009/070392

(56) References cited:
- US-A1- 2007 037 173
- US-B2- 6 558 662
- US-B2- 7 029 652
- US-B2- 7 223 596
- ULMER A ET AL: "Immunomagnetic Enrichment, Genomic Characterization, and Prognostic Impact of Circulating Melanoma Cells", CLINICAL CANCER RESEARCH, vol. 10, no. 2, 15 January 2004 (2004-01-15), pages 531-537, XP55008451, ISSN: 1078-0432, DOI: 10.1158/1078-0432.CCR-0424-03
- MEDIC S ET AL: "Molecular markers of circulating melanoma cells", PIGMENT CELL RESEARCH, vol. 20, no. 2, 1 April 2007 (2007-04-01), pages 80-91, XP009140695, ISSN: 0893-5785, DOI: 10.1111/J.1600-0749.2006.00356.X [retrieved on 2006-12-20]
- RAO CHANDRA ET AL: "Automated enumeration and characterization of circulating melanoma cells in blood", PROCEEDINGS OF THE ANNUAL MEETING OF THE AMERICAN ASSOCIATION FOR CANCER RESEARCH, vol. 49, 1 April 2008 (2008-04-01), page 874, XP009143620, ISSN: 0197-016X
- STEEN S ET AL: "Circulating tumor cells in melanoma: a review of the literature and description of a novel technique", PROC (BAYL UNIV MED CENT), vol. 21, no. 2, April 2008 (2008-04), pages 127-132, XP55007083,

## Description

### Background

### Field of the Invention

The invention relates generally to monitoring and assessing disease progression in cancer patients, based on the presence of morphologically intact circulating melanoma cells (CMC's) in blood. More specifically, methods, reagents and apparatus are described for assessing circulating melanoma cells in patients. Circulating melanoma cells are determined by highly sensitive methodologies established for isolating and imaging 1 or 2 circulating tumor cells in approximately 5 to 50 ml of peripheral blood. The level of the tumor cell number and an increase in tumor cell number provides a means to monitor patients with metastatic melanoma.

### Background Art

Treatment of advanced melanoma is complicated by its heterogeneous histopathology and changes in make-up that accumulates during tumor progression. The enumeration and characterization of circulating tumor cells in patients with either metastatic breast or colorectal cancer has been shown to provide independent prognostic and predictive information that is clinically significant and can be used to monitor patient management.

Circulating tumor cells (CTC's) have been shown to be a critical link between primary cancer, a disease stage at which cure is possible, and metastatic disease, which continues to be the leading cause of death for most malignancies. Clinical studies have shown that CTC's are a powerful prognostic and predictive biomarker in metastatic breast cancer, and similar findings have been reported in prostate cancer and colorectal cancer. These data show that CTC's are representative of the underlying biology driving metastatic cancer and suggest that further cellular and molecular analyses of these cells can reveal new insights into molecular regulation of metastasis and response to therapy.

Research on the role of CTC's in metastasis and expansion of their use as a biomarker in pharmacokinetic and pharmacodynamic studies has been limited to the clinical phase of drug development. It is generally accepted that most cancer patients are not killed by their primary tumor, but they succumb instead to metastases: multiple widespread tumor colonies established by malignant cells that detach themselves from the original tumor and travel through the body, often to distant sites. The most successful therapeutic strategy in cancer is early detection and surgical removal of the tumor while still organ confined. Early detection of cancer has proven feasible for some cancers, particularly where appropriate diagnostic tests exist such as PAP smears in cervical cancer, mammography in breast cancer, and serum prostate specific antigen (PSA) in prostate cancer. However, many cancers detected at early stages have established micrometastases prior to surgical resection. Thus, early and accurate determination of the cancer's malignant potential is important for selection of proper therapy.

If a primary tumor is detected early enough, it can often be eliminated by surgery, radiation, or chemotherapy or some combination of those treatments. Unfortunately, the metastatic colonies are difficult to detect and eliminate and it is often impossible to treat all of them successfully. Therefore, metastasis can be considered the conclusive event in the natural progression of cancer. Moreover, the ability to metastasize is a property that uniquely characterizes a malignant tumor.

Increased HER-2/neu results in decreased response to hormone therapy, and is a significant prognostic factor in predicting responses to hormone receptor-positive metastatic breast cancer. Thus in malignancies where the HER-2/neu oncogene product is associated, methods have been described to monitor therapy or assess risks based on elevated levels (US 5,876,712). However, the base levels during remission, or even in healthy normals, are relatively high and may overlap with concentrations found in patients, thus requiring multiple testing and monitoring to establish patient-dependent baseline and cut-off levels.

In prostate cancer, PSA levels in serum have proven to be useful in early detection. When used with a follow-up physical examination and biopsy, the PSA test has improved detection of prostate cancer at an early stage when it is best treated.

Detection of intact tumor cells in blood provides a direct link to recurrent metastatic disease in cancer patients who have undergone resection of their primary tumor. Unfortunately, the same spreading of malignant cells continues to be missed by conventional tumor staging procedures. Recent studies have shown that the presence of a single carcinoma cell in the bone marrow of cancer patients is an independent prognostic factor for metastatic relapse (Diel IJ, Kaufman M, Goerner R, Costa SD, Kaul S, Bastert G. Detection of tumor cells in bone marrow of patients with primary breast cancer: a prognostic factor for distant metastasis. J Clin Oncol, 10:1534-1539, 1992). But these invasive techniques are deemed undesirable or unacceptable for routine or multiple clinical assays compared to detection of disseminated epithelial tumor cells in blood.

An alternative approach incorporates immunomagnetic separation technology and provides greater sensitivity and specificity in the unequivocal detection of intact circulating cancer cells. This simple and sensitive diagnostic tool, as described (US6,365,362; US6,551,843; US6,623,982; US6,620,627; US6,645,731; WO 02/077604; WO03/065042; WO 03/019141; and Ulmer A, Schmidt-Kittler O, Fischer J, Ellwanger U, Rassner G, Riethmüller G, Fierlbeck G, Klein CA. Immunomagnetic enrichment, genomic characterization, and prognostic impact of circulating melanoma cells. Clin Cancer Res, 10:531-537, 2004) is used in the present invention to provide a preclinical animal model to enumerate CTC's.

Using this diagnostic tool, a blood sample from a cancer patient (WO 03/018757) is incubated with magnetic beads, coated with antibodies directed against an epithelial cell surface antigen as for example EpCAM. After labeling with anti-EpCAM-coated magnetic nanoparticles, the magnetically labeled cells are then isolated using a magnetic separator. The immunomagnetically enriched fraction is further processed for downstream immunocytochemical analysis or image cytometry, for example, in the CellTracks^{®} System (Veridex LLC, NJ). The magnetic fraction can also be used for downstream immunocytochemical analysis, RT-PCR, PCR, FISH, flowcytometry, or other types of image cytometry.

The CellTracks® System utilizes immunomagnetic selection and separation to highly enrich and concentrate any epithelial cells present in whole blood samples. The captured cells are detectably labeled with a leukocyte specific marker and with one or more tumor cell specific fluorescent monoclonal antibodies to allow identification and enumeration of the captured CTC's as well as unequivocal instrumental or visual differentiation from contaminating non-target cells. This assay allows tumor cell detection even in the early stages of low tumor mass. The embodiment of the present invention is not limited to the CellTracks® System.

Currently available technology has not demonstrated a consistently reliable means for repetitively monitoring CMC's in assessing metastatic melanoma cancer progression. Thus, there is a clear need for accurate detection of cancer cells with metastatic potential, not only in melanoma but in metastatic cancers in general. Moreover, this need is accentuated by the need to select the most effective therapy for a given patient.

The inability to repetitively monitor CMC's in melanoma and other cancers has restricted their analysis obtained from blood draws. As a consequence, the studies of disease progression characteristics such as temporal changes in CMC's during tumor progression and related therapy as not been established. However, using this technology to assay CMC's would permit integration of CMC assessments into pre-clinical as well as clinical studies. Further characterization of specific molecular markers on these cells would permit early development of "companion" diagnostic assays for targeted therapies, which would accelerate translation of new assay protocols into clinical trials in patients and ultimately into clinical practice.

### Summary of the Invention

The present invention provides an automated method as defined in claim 1 for isolating enriching, and analyzing circulating melanoma cells (CMC's) in the blood of patients with melanoma, incorporating clinical analysis tools such as the CellTracks® System, and is based upon the absolute number, change, or combinations of both of circulating epithelial cells in patients with metastatic cancer. The system immunomagnetically concentrates epithelial cells, fluorescently labels the cells, identifies and quantifies CMC's for positive enumeration in melanoma.

### Brief Description of the Drawings

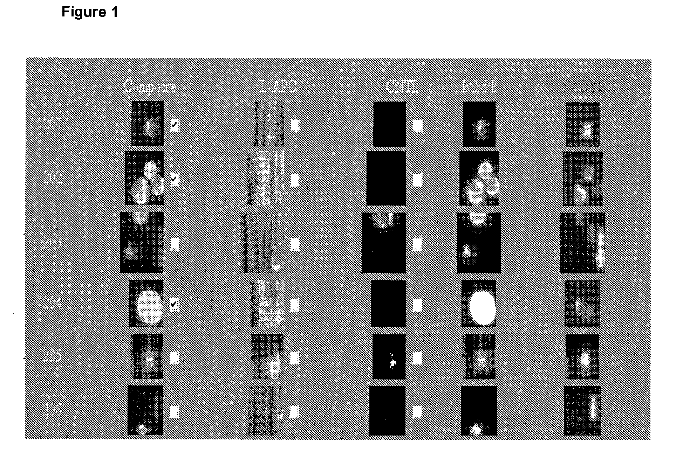

**Figure 1****:** CellTracks® fluorescent analysis profile used to confirm objects captured as human tumor cells. Check marks signify a positive tumor cell based on the composite image. Composite images are derived from the positive selection for Epithelial Cell Marker (EC-PE) and for the nuclear dye (NADYE). A negative selection is also needed for the leukocyte marker (L-APC) and for control (CNTL).

### Detailed Description of the Invention

While any effective mechanism for isolating, enriching, and analyzing CTC's in blood is appropriate, one method for collecting circulating tumor cells combines immunomagnetic enrichment technology, immunofluorescent labeling technology with an appropriate analytical platform after initial blood draw. The associated test has been shown to have the sensitivity and specificity to detect these rare cells in a sample of whole blood and to investigate their role in the clinical course of the disease in malignant tumors of epithelial origin. From a sample of whole blood, rare cells are detected with a sensitivity and specificity to allow them to be collected and used in modeling disease progression in an animal model.

Circulating tumor cells (CTC's) have been shown to exist in the blood in detectable amounts. This created a tool to investigate the significance of cells of epithelial origin in the peripheral circulation of cancer patients (Racila E., Euhus D., Weiss A.J., Rao C., McConnell J., Terstappen L.W.M.M. and Uhr J.W., Detection and characterization of carcinoma cells in the blood, Proc. Natl. Acad. Sci. USA, 95:4589-4594 (1998)). This study demonstrated that these blood-borne cells might have a significant role in the pathophysiology of cancer. Having a detection sensitivity of 1 epithelial cell per 5 ml of patient blood, the assay incorporated immunomagnetic sample enrichment and fluorescent monoclonal antibody staining followed by flowcytometry for a rapid and sensitive analysis of a sample.

The CellSearch™ System (Veridex LLC, NJ) previously has been used to isolate and enumerate circulating epithelial tumor cells from human blood samples. This is an automated system that enriches for epithelial cells using antibodies to epithelial-cell adhesion molecule coupled to magnetic beads. Isolated cells then are stained with the fluorescent nucleic acid dye 4,2-diamidino-2-phenylindole dihydrochloride (DAPI) to identify nucleated cells. Recovered cells subsequently are stained with fluorescently labeled monoclonal antibodies to CD45 (APC channel) and cytokeratin 8, 18, 19 (PE channel) to distinguish epithelial cells from leukocytes. Nucleated epithelial cells then are quantified as circulating tumor cells. There is an additional fluorescence channel for FITC that is not part of the standard CellSearch™ assay and may be used for further characterization of tumor cells.

As shown in the example, the assay was further configured to an image cytometric analysis such that the immunomagnetically enriched sample is analyzed by the CellTracks® System. This is a fluorescence-based microscope image analysis system, which in contrast with flowcytometric analysis permits the visualization of events and the assessment of morphologic features to further identify objects.

CD146 (also know as MUC18, MCAM, Mel-CAM and S-Endo-1) is a transmembrane glycoprotein possessing a limited tissue distribution, including endothelial cells, smooth muscle cells, follicular dendritic cells, melanoma cells, and a subpopulation of activated T lymphocytes.

Ki-67 (also know as antigen identified by monoclonal antibody Ki-67 or MK167 is a cellular marker for proliferation and is associated with cell proliferation. During interphase, the Ki-67 antigen can be detected within the cell nucleus, whereas in mitosis most of the protein is relocated to the surface of the chromosomes. Ki-67 protein is present during all active phases of the cell cycle (G1, S, G2, and mitosis), but is absent in resting cells (Go). It is generally used as a marker to determine the growth fraction of a cell population.

The CellTracks® AutoPrep® System and CellTracks® Analyzer II were used to fully automate the capture and detection of CMCs. In the CMC assay, magnetic particles conjugated to antibody specific for the melanoma cell adhesion molecule (CD146) are used to capture melanoma cells from 7.5 mL of blood. The enriched CMC's are then stained with the nucleic acid dye DAPI, and a monoclonal antibody conjugated to PE specific for the High Molecular Weight Melanoma Associated Antigen. The assay also contains APC conjugated monoclonal antibodies to CD45 and CD34 to exclude co-purified leukocytes and circulating endothelial cells, respectively. In addition, FITC labeled anti-Ki67 was added to determine the proportion of CMC in cell cycle (G1, S, G2 or M phase) while in circulation. The enriched and stained CMC's were magnetically mounted within a CellTracks cartridge and scanned using the CellTracks Analyzer II. Individual images of cells were presented to the operator for review, and scored as CMC's, based on fluorescence and cell morphology. The CMC assay consistently recovered >65% of the melanoma cells from the cell line SK-MEL28 when spiked into 7.5 mL of blood from healthy donors. The assay was linear over the tested range of from 1 to 1200 melanoma cells/7.5ml(r² of 0.999, slope 0.74, intercept 6.8). The assay was validated using blood from healthy donors (n=60) and patients with metastatic melanoma (n=71). In 7.5 mL blood from normal donors, 0 CMC's were detected in 57/60 (95%). One cell was stained as a CMC in 3/60 (5%) normal donors, but these cells typically had a characteristic endothelial cell morphology, and were Ki67 negative. In melanoma patients, CMC's ranged from 0 to 8000 /7.5mL blood. One or more CMC's were detected in 39% of the patients, ≥ 2 in 25%, ≥ 5 in 8%, ≥ 10 in 4% and ≥ 100 in 3%. Surprisingly, 30 to100% (mean 84%) of the CMC's were Ki67 positive suggesting a high proportion of melanoma cells shed into blood are actively dividing. This automated CMC assay is a useful monitoring device for patients with metastatic melanoma, assessing prognosis, or possibly as a tool for evaluating biomarkers, targets, and potential treatments in this difficult and aggressive disease.

### Example

### Enumeration of circulating cytokeratin positive cells

The CellTracks® System refers to an automated fluorescence microscopic system for automated enumeration of isolated cells from blood. The system contains an integrated computer controlled fluorescence microscope and automated stage with a magnetic yoke assembly that will hold a disposable sample cartridge. The magnetic yoke is designed to enable ferrofluid-labeled candidate tumor cells within the sample chamber to be magnetically localized to the upper viewing surface of the sample cartridge for microscopic viewing. Software presents suspect cancer cells, labeled with antibodies to cytokeratin and having epithelial origin, to the operator for final selection..

While isolation of tumor cells for the CellTracks® System can be accomplished by any means known in the art, the invention uses immunomagentic enrichment for isolating tumor cells from a biological sample. Epithelial cell-specific magnetic particles are added and incubated for 20 minutes. After magnetic separation, the cells bound to the immunomagnetic-linked antibodies are magnetically held at the wall of the tube. Unbound sample is then aspirated and an isotonic solution is added to resuspend the sample. A nucleic acid dye, monoclonal antibodies to cytokeratin (a marker of epithelial cells) and CD 45 (a broad-spectrum leukocyte marker) are incubated with the sample. After magnetic separation, the unbound fraction is again aspirated and the bound and labeled cells are resuspended in 0.2 ml of an isotonic solution. The sample is suspended in a cell presentation chamber and placed in a magnetic device whose field orients the magnetically labeled cells for fluorescence microscopic examination in the CellTracks® System. Cells are identified automatically in the CellTracks® System and candidate circulating tumor cells presented to the operator for checklist enumeration. An enumeration checklist consists of predetermined morphologic criteria constituting a complete cell.

Cytokeratin positive cells are isolated by immunomagnetic enrichment using a 7.5 ml sample of whole blood from humans. Epithelial cell-specific immunomagnetic fluid is added and incubated for 20 minutes. After magnetic separation for 20 minutes, the cells bound to the immunomagnetic-linked antibodies are magnetically held at the wall of the tube. Unbound sample is then aspirated and an isotonic solution is added to resuspend the sample. A nucleic acid dye, monoclonal antibodies to cytokeratin (a marker of epithelial cells) and CD 45 (a broad-spectrum leukocyte marker) are incubated with the sample for 15 minutes. After magnetic separation, the unbound fraction is again aspirated and the bound and labeled cells are resuspended in 0.2 ml of an isotonic solution. The sample is suspended in a cell presentation chamber and placed in a magnetic device whose field orients the magnetically labeled cells for fluorescence microscopic examination in the CellTracks® System. Cells are identified automatically in the CellTracks® System; control cells are enumerated by the system, whereas the candidate circulating tumor cells are presented to the operator for enumeration using a checklist as shown in Figure 1.

## Claims

1. A method for isolating, enriching, and analyzing circulating melanoma cells in a blood sample which comprises a mixed cell population suspected of containing circulating melanoma cells and which has been isolated from a patient with metastatic melanoma disease, the method comprising:
a) enriching a fraction of the sample using magnetic particles conjugated to an antibody specific for the melanoma cell adhesion molecule (CD146), the fraction containing the circulating melanoma cells;
b) confirming structural integrity of the rare circulating melanoma cells to be intact, wherein said structural integrity is determined by the nucleic acid dye DAPI, and a Phycoerythrin (PE) conjugated monoclonal antibody for High Molecular Weight Melanoma Associated Antigen wherein said structural integrity is further confirmed by exclusion of co-enriched leukocytes and circulating endothelial cells using Allophycocyanin (APC) conjugated monoclonal CD45 and CD34 antibodies;
c) adding FITC labeled anti-Ki67 to determine the proportion of circulating melanoma cells in active cell cycle.

## Patentansprüche

1. Verfahren zum Isolieren, Anreichern und Analysieren zirkulierender Melanomzellen in einer Blutprobe, die eine gemischte Zellpopulation, die vermutlich zirkulierende Melanomzellen enthält, umfasst und die aus einem Patienten mit metastatischer Melanomkrankheit isoliert wurde, wobei man bei dem Verfahren:
a) eine Fraktion der Probe unter Verwendung von an einen für das Melanomzelladhäsionsmolekül (CD146) spezifischen Antikörper konjugierten magnetischen Partikeln anreichert, wobei die Fraktion die zirkulierenden Melanomzellen enthält;
b) bestätigt, dass die strukturelle Integrität der seltenen zirkulierenden Melanomzellen intakt ist, wobei die strukturelle Integrität mit dem Nukleinsäurefarbstoff DAPI und einem an Phycoerythrin (PE) konjugierten monoklonalen Antikörper für das High Molecular Weight Melanoma Associated Antigen bestimmt wird, wobei die strukturelle Integrität ferner mittels Ausschluss mitangereicherter Leukozyten und zirkulierender Endothelzellen unter Verwendung der an Allophycocyanin (APC) konjugierten monoklonalen Antikörper CD45 und CD34 bestätigt wird;
c) FITC-markiertes Anti-Ki67 hinzugibt, um den Anteil zirkulierender Melanomzellen im aktiven Zellzyklus zu bestimmen.

## Revendications

1. Procédé pour isoler, enrichir, et analyser des cellules de mélanome circulantes dans un échantillon de sang qui comprend une population de cellules mixte suspecté de contenir des cellules de mélanome circulantes qui a été isolé à partir d'un patient atteint de mélanome métastasique, le procédé comprenant :
a) l'enrichissement d'une fraction de l'échantillon au moyen de particules magnétiques conjuguées à un anticorps spécifique pour la molécule d'adhésion de cellule de mélanome (CD146), la fraction contenant les cellules de mélanome circulantes ;
b) la confirmation que l'intégrité structurale des cellules de mélanome circulantes rares est intacte, ladite intégrité structurale étant déterminée par le colorant d'acide nucléique DAPI, et un anticorps monoclonal pour l'antigène associé au mélanome de poids moléculaire élevé conjugué à la phycoérythrine (PE), ladite intégrité structurale étant confirmée plus avant par l'exclusion de leucocytes et de cellules endothéliales circulantes co-enrichis au moyen d'anticorps pour CD45 et CD34 monoclonaux conjugués à l'allophycocyanine (APC) ;
c) l'ajout d'anti-Ki67 marqué avec FITC afin de déterminer la proportion de cellules de mélanome circulantes en cycle cellulaire actif.
